# EUROPEAN PATENT APPLICATION

(11) **EP 2 604 690 A1**
(43) Date of publication of application: **19.06.2013**
(21) Application number: 11193873.4
(22) Date of filing: 15.12.2011
(51) Int. Cl.: C12N 15/113

(54) **MicroRNAs and uses thereof**

(71) Applicant: Oncostamen S.r.l., 00145 Roma (IT)
(72) Inventor: Coppola, Valeria, 00182 Roma (IT); De Maria Marchiano, Ruggero, 00161 Roma (IT); Fiori, Micol Eleonora, 00197 Roma (IT); Garaci, Enrico, 00198 Roma (IT); Todaro, Matilde, 90151 Palermo (IT)
(74) Representative: Predazzi, Valentina

(57) **Abstract**

The invention relates to molecular targets and their use to counteract tumors. Naturally occurring microRNAs that regulate human oncogenes and methods of use thereof are described. Suitable nucleic acids for use in the methods and compositions described herein include, but are not limited to, pri-miRNA, pre-miRNA, mature miRNA or fragments of variants thereof that retain the biological activity of the mature miRNA and DNA encoding a pri-miRNA, pre-miRNA, mature miRNA, fragments or variants thereof, or regulatory elements of the miRNA. The here claimed approach is efficacious also on Cancer Stem Cells.

SEQUENCE LISTING

<110>  Oncostamen srl

<120>  Micrornas and uses thereof

<130>  E0083574

<160>  47

<170>  PatentIn version 3.5

<210>  1
<211>  22
<212>  RNA
<213>  Homo sapiens

<400>  1
uuuggucccc uucaaccagc ug                                                22

<210>  2
<211>  22
<212>  RNA
<213>  Homo sapiens

<400>  2
uuuggucccc uucaaccagc ua                                                22

<210>  3
<211>  22
<212>  RNA
<213>  Homo sapiens

<400>  3
cuuggcaccu agcaagcacu ca                                                22

<210>  4
<211>  23
<212>  RNA
<213>  Homo sapiens

<400>  4
ucccccaggu gugauucuga uuu                                               23

<210>  5
<211>  26
<212>  RNA
<213>  Homo sapiens

<400>  5
gugagggcau gcaggccugg augggg                                            26

<210>  6
<211>  23
<212>  RNA
<213>  Homo sapiens

<400>  6
cacucagccu ugagggcacu uuc                                               23

<210> 7
<211> 22
<212> RNA
<213> Homo sapiens

<400> 7
ucugggcaac aaagugagac cu 22


<210> 8
<211> 22
<212> RNA
<213> Homo sapiens

<400> 8
uucaccaccu ucuccaccca gc 22


<210> 9
<211> 22
<212> RNA
<213> Homo sapiens

<400> 9
cuauacaacc uacugccuuc cc 22


<210> 10
<211> 23
<212> RNA
<213> Homo sapiens

<400> 10
ugucugcccg caugccugcc ucu 23


<210> 11
<211> 22
<212> RNA
<213> Homo sapiens

<400> 11
agaaggaaau ugaauucauu ua 22


<210> 12
<211> 22
<212> RNA
<213> Homo sapiens

<400> 12
uccgagccug ggucucccuc uu 22


<210> 13
<211> 23
<212> RNA
<213> Homo sapiens

<400> 13
ugugcuugcu cgucccgccc gca 23


<210> 14

<211>  20
<212>  RNA
<213>  Homo sapiens

<400>  14
cgugccaccc uuuuccccag                                                    20


<210>  15
<211>  22
<212>  RNA
<213>  Homo sapiens

<400>  15
uccauuacac uacccugccu cu                                                 22


<210>  16
<211>  22
<212>  RNA
<213>  Homo sapiens

<400>  16
acuccagccc cacagccuca gc                                                 22


<210>  17
<211>  22
<212>  RNA
<213>  Homo sapiens

<400>  17
uuagggcccu ggcuccaucu cc                                                 22


<210>  18
<211>  22
<212>  RNA
<213>  Homo sapiens

<400>  18
uucccuuugu cauccuaugc cu                                                 22


<210>  19
<211>  23
<212>  RNA
<213>  Homo sapiens

<400>  19
caacggaauc ccaaaagcag cug                                                23


<210>  20
<211>  22
<212>  RNA
<213>  Homo sapiens

<400>  20
aggcggggcg ccgcgggacc gc                                                 22


<210>  21
<211>  22
<212>  RNA

<213>  Homo sapiens

<400>  21
cacgcucaug cacacaccca ca                                              22


<210>  22
<211>  23
<212>  RNA
<213>  Homo sapiens

<400>  22
ucucacacag aaaucgcacc cgu                                             23


<210>  23
<211>  22
<212>  RNA
<213>  Homo sapiens

<400>  23
uauugcacuu gucccggccu gu                                              22


<210>  24
<211>  22
<212>  RNA
<213>  Homo sapiens

<400>  24
uccuguacug agcugccccg ag                                              22


<210>  25
<211>  22
<212>  RNA
<213>  Homo sapiens

<400>  25
ucucccaacc cuuguaccag ug                                              22


<210>  26
<211>  22
<212>  RNA
<213>  Homo sapiens

<400>  26
cugugcgugu gacagcggcu ga                                              22


<210>  27
<211>  21
<212>  RNA
<213>  Homo sapiens

<400>  27
ggcuagcaac agcgcuuacc u                                               21


<210>  28
<211>  23
<212>  RNA
<213>  Homo sapiens

<400>  28
guccaguuuu cccaggaauc ccu                                              23

<210>  29
<211>  23
<212>  RNA
<213>  Homo sapiens

<400>  29
cucucaccac ugcccuccca cag                                              23

<210>  30
<211>  21
<212>  RNA
<213>  Homo sapiens

<400>  30
aagccugccc ggcuccucgg g                                                21

<210>  31
<211>  21
<212>  RNA
<213>  Homo sapiens

<400>  31
agggcccccc cucaauccug u                                                21

<210>  32
<211>  22
<212>  RNA
<213>  Homo sapiens

<400>  32
uggaauguaa ggaagugugu gg                                               22

<210>  33
<211>  22
<212>  RNA
<213>  Homo sapiens

<400>  33
acuggacuua gggucagaag gc                                               22

<210>  34
<211>  21
<212>  RNA
<213>  Homo sapiens

<400>  34
gugcauugua guugcauugc a                                                21

<210>  35
<211>  22
<212>  RNA
<213>  Homo sapiens

<400>  35
ucucugggcc ugugucuuag gc                                               22

<210>  36
<211>  23
<212>  RNA
<213>  Homo sapiens

<400>  36
cucuugaggg aagcacuuuc ugu                                              23


<210>  37
<211>  22
<212>  RNA
<213>  Homo sapiens

<400>  37
agaucagaag gugauugugg cu                                               22


<210>  38
<211>  20
<212>  RNA
<213>  Homo sapiens

<400>  38
ucguuugccu uuuucugcuu                                                  20


<210>  39
<211>  22
<212>  RNA
<213>  Homo sapiens

<400>  39
caguuaucac agugcugaug cu                                               22


<210>  40
<211>  22
<212>  RNA
<213>  Homo sapiens

<400>  40
caaaacuggc aauuacuuuu gc                                               22


<210>  41
<211>  21
<212>  RNA
<213>  Homo sapiens

<400>  41
aaagugcuuc cuuuuagagg g                                                21


<210>  42
<211>  22
<212>  RNA
<213>  Homo sapiens

<400>  42
gccugcuggg guggaaccug gu                                               22

<210> 43
<211> 22
<212> RNA
<213> Homo sapiens

<400> 43
auaagacgag caaaaagcuu gu 22


<210> 44
<211> 22
<212> RNA
<213> Homo sapiens

<400> 44
auaagacgaa caaaagguuu gu 22


<210> 45
<211> 22
<212> RNA
<213> Homo sapiens

<400> 45
ucagcaaaca uuuauugugu gc 22


<210> 46
<211> 22
<212> RNA
<213> Homo sapiens

<400> 46
ucaguaaaug uuuauuagau ga 22


<210> 47
<211> 22
<212> RNA
<213> Homo sapiens

<400> 47
uagguaguuu ccuguuguug gg 22

## Description

The invention relates to molecular targets and their use to counteract tumors. Naturally occurring microRNAs that regulate human oncogenes and methods of use thereof are described. Suitable nucleic acids for use in the methods and compositions described herein include, but are not limited to, pri-miRNA, pre-miRNA, mature miRNA or fragments of variants thereof that retain the biological activity of the mature miRNA and DNA encoding a pri-miRNA, pre-miRNA, mature miRNA, fragments or variants thereof, or regulatory elements of the miRNA. The here claimed approach is efficacious also on Cancer Stem Cells.

### Background

Cancer is a group of diseases characterized by uncontrolled growth and spread of abnormal cells. Cancer is caused by both external factors (tobacco, chemicals, radiation, and infections organisms) and internal factors (inherited mutations, hormones, immune conditions, and DNA damage). These factors may act together or sequentially to initiate and/or promote carcinogenesis. Cancer causes 1 of every 4 deaths and it is the leading cause of death in people under age 85 in the United States. Nearly half of all men and a little over one third of all women in the U.S. will develop cancer during their lifetimes. Today, millions of people are living with cancer or have had cancer. The sooner a cancer is found and treatment begins, the better are the chances for living for many years.

Misregulation of genes that control cell fate determination often contributes to cancer. Such altered genes are known as oncogenes. Oncogenes are called proto-oncogenes when they are normal (i.e., not mutated). Proto-oncogenes encode components of the cell's normal growth-control pathway. Some of these components are growth factors, receptors, signaling enzymes, and transcription factors.

In 2001, several groups used a novel cloning method to isolate and identify a large group of "microRNAs" (miRNAs) from C. elegans, Drosophila, and humans (Lagos-Quintana et al., 2001; Lau et al., 2001; Lee and Ambros, 2001). Several hundreds of miRNAs have been identified in plants and animals- including humans-which do not appear to have endogenous siRNAs (small interference RNA). Thus, while similar to siRNAs, miRNAs are nonetheless distinct. miRNAs are 21-24 nucleotide (nt) duplex RNAs that are created from precursor transcripts by subsequent processing steps mediated by members of the RNAseIII family, Drosha and Dicer. One of the two strands is incorporated into the active sites of the Argonaute family of proteins, where it serves as a guide for Watson-Crick base pairing with complementary sequences in target messenger RNAs (mRNAs).

Animal miRNAs are initially transcribed as part of one arm of an ∼80 nucleotide RNA stem-loop that in turn forms part of a several hundred nucleotides long miRNA precursor termed a primary miRNA (pri-miRNA)s. The double-stranded RNA structure of the hairpins in a pri-miRNA is recognized by a nuclear protein known as DGCR8. DGCR8 associates with the enzyme Drosha to form a complex where DGCR8 orients the catalytic RNase III domain of Drosha to liberate hairpins from pri-miRNAs. The product resulting is often termed as a pre-miRNA (precursor-miRNA). A single pri-miRNA may contain from one to six precursor-miRNA. pre-miRNA hairpins are exported from the nucleus in a process involving the nucleocytoplasmic shuttle Exportin-5. This protein, a member of the karyopherin family, recognizes a two-nucleotide overhang left by the RNase III enzyme Drosha at the 3' end of the pre-miRNA hairpin. In the cytoplasm, the pre-miRNA hairpin is cleaved by the RNase III enzyme Dicer. This endoribonuclease interacts with the 3' end of the hairpin and cuts away the loop joining the 3' and 5' arms, yielding an imperfect miRNA:miRNA* duplex about 22 nucleotides in length.

In mammals, the majority of miRNAs guide the RNA-induced silencing complex (RISC) to the 3' untranslated regions (UTRs) of mRNA targets, with the consequence that translation of the target mRNAs is inhibited. miRNA molecules interrupt translation through precise or imprecise base-pairing with their targets. Although either strand of the duplex may potentially act as a functional miRNA, only one strand is usually incorporated into the RNA-induced silencing complex (RISC) where the miRNA and its mRNA target interact.

The roughly 1000 miRNAs identified until now are thought to regulate over 30% of the expressed mRNA (Croce, C.M. Causes and consequences of microRNA disregulation in cancer. Nat Rev Genet 10, 704-714, 2009). This explains why miRNAs play roles in many fundamental biological processes such as cell proliferation, apoptosis, stress response, development and differentiation. Hence, it is not surprising that a growing number of studies describe aberrant expression of miRNAs with oncogenic potential in many human tumors (Esquela-Kerscher, A. & Slack, F.J. Oncomirs - microRNAs with a role in cancer. Nat Rev Cancer 6, 259-269, 2006). The connection between aberrant miRNA expression and tumor growth, spread and survival, suggests a new strategy to fight cancer by interfering with miRNA function. This strategy is particularly interesting, as the current technology allows for RNA-based pharmacological approaches not only in vitro, but also in vivo. Recent studies demonstrate a functional modulation of miRNA- and mRNA-levels using antagomiR-, LNA- and siRNA- treatments in mice (Ma, L., et al. Therapeutic silencing of miR-10b inhibits metastasis in a mouse mammary tumor model. Nat Biotechnol, 2010), non-human primates (Elmen, J., et al. LNA-mediated microRNA silencing in non-human primates. Nature 452, 896-899, 2008; Lanford, R.E., et al. Therapeutic silencing of microRNA-122 in primates with chronic hepatitis C virus infection. Science 327, 198-201, 2010) and even in humans (Davis, M.E., et al. Evidence of RNAi in humans from systemically administered siRNA via targeted nanoparticles. Nature, 2010). These results strongly suggest that pharmacological manipulation of miRNA expression might represent a functional instrument for innovative anti cancer therapy.

Conventional anti cancer therapies such as radiation and chemotherapy mainly aim to kill fast proliferating tumor cells by inducing DNA damage or interfering with mitosis. However, unfortunately they show only limited efficacy in the treatment of solid tumors like breast, lung and colorectal cancer. For solid malignancies these widely accepted clinical approaches may delay the progression of the disease, but very rarely lead to complete eradication of the tumor. This poor clinical outcome as well as the marked heterogeneity observed in many solid tumors can be explained by the existence of Cancer Stem Cells (CSCs). CSCs represent an undifferentiated pool of cells within the tumor mass, which have high self renewal capacities and are responsible for tumor growth and spread (Clarke, M.F., et al. Cancer stem cells--perspectives on current status and future directions: AACR Workshop on cancer stem cells. Cancer Res 66, 9339-9344, 2006). They were shown to be highly chemo- and radio-resistant and therefore are likely to be responsible for the frequent relapses after conventional tumor treatment observed in clinics. CSCs isolation and characterization has been achieved in many solid tumors on the basis of phenotypic and functional features and still represents a field of intensive investigation (Visvader, J.E. & Lindeman, G.J. Cancer stem cells in solid tumours: accumulating evidence and unresolved questions. Nat Rev Cancer 8, 755-768, 2008). The recent identification of CSCs from solid tumors has generated new perspectives for the development of cancer therapies potentially able to eradicate the entire neoplastic population.

The present study identifies a defined series of molecular targets, in particular miRNAs, involved in the proliferation of cancer cells and, preferably, of CSCs, thus offering a novel therapeutic approach in tumors.

### Figure description

*Figure 1**:* Control of the transfection efficiency of breast cancer stem cells by transfecting siRNA targeting NF-1B and subsequent determination of NF-1B mRNA 48 hours post transfection.
*Figure 2**:* Representative result of the screening for cell viability upon neutralization of miRNAs using the indicated LNA library. Dotted line: borders of statistic significance (2 SDs from the average viability). Box on the left: group of LNAs leading to a decrease of cell viability. Box on the right: group of LNA leading to an increase of cell viability.

### Detailed description:

The present invention identifies specific and new molecular target to be addressed to achieve an efficacious therapeutic outcome in tumors therapy. A genome-wide anti-miRNA screenings has been performed, systematically neutralizing the function of a given hsa-miRNA (*Homo sapiens* miRNA) in primary breast and lung cancer derived CSCs. The obtained results were then confirmed in commercially available cell lines. To neutralize more than 90% of experimentally confirmed hsa-miRNAs expressed by human cells, a comprehensive locked nucleic acids (LNA) based anti-miRNA library has been used. This library consists of LNAs targeting more than 800 miRNAs (miRCURY LNA™ microRNA inhibitor library, Human v.12.0, Exiqon). LNA-mediated miRNA neutralization has been chosen as this technology has a number of advantages compared to standard anti-miRs or antagomiRs:
- LNAs offer increased thermal stability in a duplex with the target miRNA, leading to a more efficient neutralization of the biological function of the miRNA;
- LNAs show a higher specificity and can discriminate even miRNAs differing in single nucleotides, this reduces also the risk of unwanted "off-target" effects;
- LNAs are more resistant to endo- and exonucleases, which increases the stability and efficacy in vitro and in vivo;
- LNAs targeting miR-122 have proven to be safe and already entered clinical phase II studies for the treatment of hepatitic C virus infections in humans.

By a genome-wide anti-miRNA screening, a series of miRNA to be targeted in cancer therapy via available technologies has been identified.

What we claim are hsa-miRNA to be inhibited to achieve a favorable therapeutic outcome in tumors, in particular in breast and lung tumors. The identified hsa-miRNA are referred to by using the standard nomenclature system and they are: hsa-miR-133a (SEQ. ID 1)/133b (SEQ. ID 2), hsa-miR-1271 (SEQ. ID 3), hsa-miR-361-3 (SEQ. ID 4), hsa-miR-1226* (SEQ. ID 5), hsa-miR-512-5p (SEQ. ID 6), hsa-miR-1285 (SEQ. ID 7), hsa-miR-197 (SEQ. ID 8), hsa-miR-Let-7b* (SEQ. ID 9), hsa-miR-346 (SEQ. ID 10), hsa-miR-1252 (SEQ. ID 11), hsa-miR-615-3p (SEQ. ID 12), hsa-miR-636 (SEQ. ID 13), hsa-miR-1227 (SEQ. ID 14), hsa-miR-885-5p (SEQ. ID 15), hsa-miR-766 (SEQ. ID 16), hsa-miR-1296 (SEQ. ID 17), hsa-miR-204 (SEQ. ID 18), hsa-miR-191 (SEQ. ID 19), hsa-miR-663 (SEQ. ID 20), hsa-miR-574-3p (SEQ. ID 21), hsa-miR-342-3p (SEQ. ID 22), hsa-miR-92a (SEQ. ID 23), hsa-miR-486-5p (SEQ. ID 24), hsa-miR-150 (SEQ. ID 25), hsa-miR-210 (SEQ. ID 26), hsa-miR-621 (SEQ. ID 27), hsa-miR-145 (SEQ. ID 28), hsa-miR-1229 (SEQ. ID 29), hsa-miR-596 (SEQ. ID 30), hsa-miR-296-5p (SEQ. ID 31).

The inhibition of the activity of hsa-miR-133a/133b, hsa-miR-1271, hsa-miR-361-3, hsa-miR-1226*, hsa-miR-512-5p, hsa-miR-1285, hsa-miR-197, hsa-miR-Let-7b*, hsa-miR-346, hsa-miR-1252, hsa-miR-615-3p, hsa-miR-636, hsa-miR-1227, hsa-miR-885-5p, hsa-miR-766, hsa-miR-1296, hsa-miR-204, hsa-miR-191, hsa-miR-663, hsa-miR-574-3p, hsa-miR-342-3p, hsa-miR-92a, hsa-miR-486-5p, hsa-miR-150, hsa-miR-210, hsa-miR-621, hsa-miR-145, hsa-miR-1229, hsa-miR-596, hsa-miR-296-5p is obtained by one of the available technologies, preferably selected from a locked nucleic acid (LNA) oligo, a Morpholino oligo or a 2'-O-methyl RNA oligo. Moreover, said at least one miRNA is silenced by a complementary antagomirR or specific steric-blocking oligo that inhibit the maturation of said miRNA. In a second embodiment, transcriptional inhibition of at least one of said miRNAs is obtained through siRNA directed against the genomic miRNA promoter, thus inducing chromatin changes, such as histone acetylation. In a third embodiment, the amount of processed miRNA available for target gene silencing is reduced with siRNA directed against the miRNA biogenesis components such as Drosha.

Said inhibition of at least one of a hsa-miRNA selected from hsa-miR-133a/133b, hsa-miR-1271, hsa-miR-361-3, hsa-miR-1226*, hsa-miR-512-5p, hsa-miR-1285, hsa-miR-197, hsa-miR-Let-7b*, hsa-miR-346, hsa-miR-1252, hsa-miR-615-3p, hsa-miR-636, hsa-miR-1227, hsa-miR-885-5p, hsa-miR-766, hsa-miR-1296, hsa-miR-204 has a cytotoxic/cytostatic effect on both cancer stem cells and differentiated carcinoma cells, preferably from breast or lung tumors.

Said inhibition of at least one of the hsa-miRNA selected from hsa-miR-574-3p, hsa-miR-342-3p, hsa-miR-92a, hsa-miR-486-5p, hsa-miR-150, hsa-miR-210, hsa-miR-621, hsa-miR-145, hsa-miR-1229, hsa-miR-596, hsa-miR-191, hsa-miR-296-5p, hsa-miR-663 leads to a cytotoxic/cytostatic effect specifically on differentiated cancer cells, preferably the effect is achieved on breast tumor epithelial cells, non-small-cell lung carcinoma cells, lung tumor epithelial cells.

In a further embodiment of the present invention, we claim a combination with conventional chemotherapeutic drugs of at least one of the inhibitors of miRNA hsa-miR-133a/133b, hsa-miR-1271, hsa-miR-361-3, hsa-miR-1226*, hsa-miR-512-5p, hsa-miR-1285, hsa-miR-197, hsa-miR-Let-7b*, hsa-miR-346, hsa-miR-1252, hsa-miR-615-3p, hsa-miR-636, hsa-miR-1227, hsa-miR-885-5p, hsa-miR-766, hsa-miR-1296, hsa-miR-204, hsa-miR-191, hsa-miR-574-3p, hsa-miR-342-3p, hsa-miR-92a, hsa-miR-486-5p, hsa-miR-150, hsa-miR-210, hsa-miR-621, hsa-miR-145, hsa-miR-1229, hsa-miR-596, hsa-miR-191, hsa-miR- hsa-296-5p, hsa-miR-663. Preferably, said chemotherapeutic drugs are paclitaxel and/or cisplatin. One of said combinations is preferably used to treat breast or lung tumors.

We further claim a set of miRNA having a tumors suppressor function. These tumors suppressors hsa-miRNA are: hsa-miR-206 (SEQ. ID 32), hsa-miR-422a (SEQ. ID 33), hsa-miR-33a (SEQ. ID 34), hsa-miR-330-5p (SEQ. ID 35), hsa-miR-526b (SEQ. ID 36), hsa-miR-383 (SEQ. ID 37), hsa-miR-1282 (SEQ. ID 38), hsa-miR-101* (SEQ. ID 39), hsa-miR-548 family (SEQ. ID 40), hsa-miR-520 family (SEQ. ID 41), hsa-miR-370 (SEQ. ID 42). The tumor suppressor activity of the above listed miRNA is in particular observed in breast cancer. A transient increased expression of at least one of said miRNA is accomplished through the introduction of a synthetic miRNA mimic into the cytoplasm that can be processed and loaded into miRNA RNA induced silencing complex (miRISC) by Dicer. This is an approach of choice for local administration in easily accessible tissues, such as lungs. In a different approach, miRNAs is expressed from RNA polymerase (Pol) III promoters as an artificial short hairpin RNA (shRNA) that bypasses Drosha processing, yet is cleaved and loaded into miRISC by Dicer. Alternatively, the entire pri-miRNA is expressed from an RNA Pol II promoter, leaving open the possibility for tissue-specific or induced ectopic miRNA expression.

Moreover, hsa-miR-548 family (SEQ. ID 40), hsa-miR-520 family (SEQ. ID 41), hsa-miR-208a (SEQ. ID 43) and hsa-miR-208b (SEQ. ID 44), hsa-miR-545-3p (SEQ. ID 45) and hsa-miR-545-5p (SEQ. ID 46), hsa-miR-196b (SEQ. ID 47) are capable to avoid the resistance of tumor cells to standard chemotherapeutic agents. Preferably, an increased functionality of hsa-miR-548 family, hsa-miR-520 family, hsa-miR-208a and hsa-miR-208b, hsa-miR-545-5p and hsa-miR-545-3p, hsa-miR-196b prevents paclitaxel resistance in breast cancer and cisplatin resistance in lung cancer.

The detailed description of the embodiments and the examples that are reported below are given by way of illustration and are not intended to limit the present invention.

### EXPERIMENTAL PART

**Table 1:** sequence of hsa-miRNAs (mature human miRNAs)whose inhibition has a cytotoxic/cytostatic effect on cancer stem cells and/or differentiated carcinoma cells.

| | | |
|---|---|---|
| SEQ. ID 1 | 133a⁽ⁱ⁾ | UUUGGUCCCCUUCAACCAGCUG |
| SEQ. ID 2 | 133b⁽ⁱ⁾ | UUUGGUCCCCUUCAACCAGCUA |
| SEQ. ID 3 | 1271 | CUUGGCACCUAGCAAGCACUCA |
| SEQ. ID 4 | 361-3p | UCCCCCAGGUGUGAUUCUGAUUU |
| SEQ.ID 5 | 1226* | GUGAGGGCAUGCAGGCCUGGAUGGGG |
| SEQ.ID 6 | 512-5p | CACUCAGCCUUGAGGGCACUUUC |
| SEQ. ID 7 | 1285 | UCUGGGCAACAAAGUGAGACCU |
| SEQ.ID 8 | 197 | UUCACCACCUUCUCCACCCAGC |
| SEQ. ID 9 | let-7b* | CUAUACAACCUACUGCCUUCCC |
| SEQ. ID 10 | 346 | UGUCUGCCCGCAUGCCUGCCUCU |
| SEQ. ID 11 | 1252 | AGAAGGAAAUUGAAUUCAUUUA |
| SEQ. ID 12 | 615-3p | UCCGAGCCUGGGUCUCCCUCUU |
| SEQ. ID 13 | 636 | UGUGCUUGCUCGUCCCGCCCGCA |
| SEQ. ID 14 | 1227 | CGUGCCACCCUUUUCCCCAG |
| SEQ. ID 15 | 885-5p | UCCAUUACACUACCCUGCCUCU |
| SEQ. ID 16 | 766 | ACUCCAGCCCCACAGCCUCAGC |
| SEQ. ID 17 | 1296 | UUAGGGCCCUGGCUCCAUCUCC |
| SEQ. ID 18 | 204 | UUCCCUUUGUCAUCCUAUGCCU |
| SEQ. ID 19 | 191 | CAACGGAAUCCCAAAAGCAGCUG |
| SEQ. ID 20 | 663 | AGGCGGGGCGCCGCGGGACCGC |
| SEQ. ID 21 | 574-3p | CACGCUCAUGCACACACCCACA |
| SEQ. ID 22 | 342-3p | UCUCACACAGAAAUCGCACCCGU |
| SEQ. ID 23 | 92a | UAUUGCACUUGUCCCGGCCUGU |
| SEQ. ID 24 | 486-5p | UCCUGUACUGAGCUGCCCCGAG |
| SEQ. ID 25 | 150 | UCUCCCAACCCUUGUACCAGUG |
| SEQ. ID 26 | 210 | CUGUGCGUGUGACAGCGGCUGA |
| SEQ. ID 27 | 621 | GGCUAGCAACAGCGCUUACCU |
| SEQ. ID 28 | 145 | GUCCAGUUUUCCCAGGAAUCCCU |
| SEQ. ID 29 | 1229 | CUCUCACCACUGCCCUCCCACAG |
| SEQ. ID 30 | 596 | AAGCCUGCCCGGCUCCUCGGG |
| SEQ. ID 31 | 296-5p | AGGGCCCCCCCUCAAUCCUGU |

| | | |
|---|---|---|
| ⁽ⁱ⁾ 133a and 133b belong to the same family. When results are indicated as 133a/133b, the reported values are the mean result of the two mature miRNAs | | |

**Table 2: sequence of hsa-miRNAs having a tumors suppressor fucntion.**

| | | |
|---|---|---|
| SEQ. ID 32 | 206 | UGGAAUGUAAGGAAGUGUGUGG |
| SEQ. ID 33 | 422a | ACUGGACUUAGGGUCAGAAGGC |
| SEQ. ID 34 | 33a | GUGCAUUGUAGUUGCAUUGCA |
| SEQ. ID 35 | 330-5p | UCUCUGGGCCUGUGUCUUAGGC |
| SEQ. ID 36 | 526b | CUCUUGAGGGAAGCACUUUCUGU |
| SEQ. ID 37 | 383 | AGAUCAGAAGGUGAUUGUGGCU |
| SEQ. ID 38 | 1282 | UCGUUUGCCUUUUUCUGCUU |
| SEQ. ID 39 | 101* | CAGUUAUCACAGUGCUGAUGCU |
| SEQ. ID 40 | miR-548 a1 (for 548 family) ⁽ⁱⁱ⁾ | CAAAACUGGCAAUUACUUUUGC |
| SEQ. ID 41 | miR-520b (for 520 family) (ⁱⁱⁱ) | AAAGUGCUUCCUUUUAGAGGG |
| SEQ. ID 42 | 370 | GCCUGCUGGGGUGGAACCUGGU |

| | | |
|---|---|---|
| ⁽ⁱⁱ⁾ when miR-548 family is indicated, the reported values are the mean result of all the mature miRNAs belonging to the family. ⁽ⁱⁱⁱ⁾ when miR-520 family is indicated, the reported values are the mean result of all the mature miRNAs belonging to the family. | | |

**Table 3: sequence of has-miRNAs capable to avoid the resistance of tumor cells to standard chemotherapeutic agents.**

| | | |
|---|---|---|
| SEQ.ID 43 | miR-208a ^{(iv)} | AUAAGACGAGCAAAAAGCUUGU |
| SEQ. ID 44 | miR-208b ^{(iv)} | AUAAGACGAACAAAAGGUUUGU |
| SEQ. ID 45 | miR-545-3p ^{(v)} | UCAGCAAACAUUUAUUGUGUGC |
| SEQ. ID 46 | miR-545-5p ^{(v)} | UCAGUAAAUGUUUAUUAGAUGA |
| SEQ. ID 47 | 196b | UAGGUAGUUUCCUGUUGUUGGG |

| | | |
|---|---|---|
| ^{(iv)} 208a and 208b belong the same family. When results are indicated as 208a/208b the reported values are the mean result of the two mature miRNAs. ^{(v)} 545-3p and 545-5p belong the same family. When results are indicated as 545-3p/545-5p the reported values are the mean result of the two mature miRNAs. | | |

### Example 1: Identification of LNAs cytotoxic/cytostatic in breast cancer stem cells, lung cancer stem cells and breast and lung cancer cell lines.

Breast cancer stem cells, lung cancer stem cells derived from patient samples and commercially available breast and lung cancer cell lines were seeded in 96 well, plates and transfected with the LNA-based anti-miR 190101-00 - miRCURY LNA™ microRNA Inhibitor Library - Human v. 12.0 using a lipofection reagent (Dharmafect 2 [Dharmacon] or, alternatively, HiPerfect [Qiagen]).

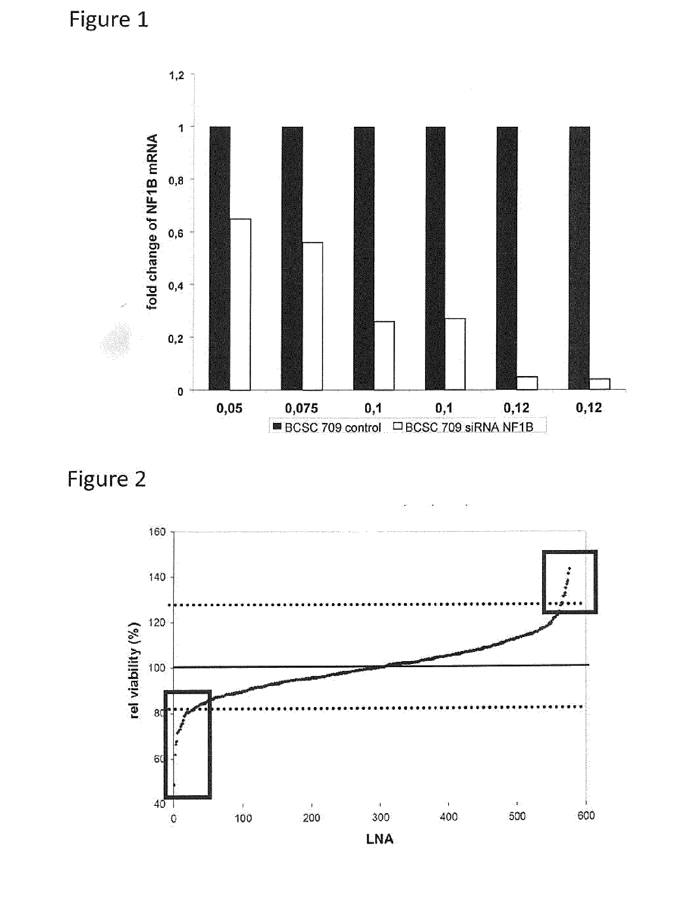

The transfection efficiency was controlled by a siRNA mediated knockdown of an indicator gene (NF-1B) followed by the analysis of NF-1B mRNA levels 48 hours post transfection using quantitative PCR. The chosen transfection conditions led to knockdown rates of 85-95% for NF-1B, indicating an efficient uptake of siRNA oligonucleotides under the screening conditions (figure 1), without signs of viability loss.

72-96 hours after transfection, cell viability was determined using an ATP-based readout (CellTiter-Glo [PROMEGA]). The relative viability was calculated by defining the average of the plates as 100% viability. This value was used to normalize the individual values obtained in the single wells.

A number of LNAs have been found to influence the viability of breast and lung cancer stem cells, as well as differentiated cell lines from both entities in both directions, leading to a decrease, or to an increase in cell number after treatment (figure 2).

18 LNAs that led to a marked decrease of cell viability in cells transfected with the inhibitor have been identified (see table 1). Most of these LNAs showed pharmacological efficiency in all cellular systems analyzed (breast cancer stem cells, differentiated breast cancer line MDA/MB231, lung cancer stem cells (LCSC136) and the lung cancer lines NIH-H460 and A549).

**Table 4: LNAs leading to a decrease in cell viability after LNA transfection. For each cancer stem cell and cell line used % of cell viability after LNA transfection is reported**

| hsa-miRNA | BCSC208 | MDA/MB231 | NIH-H460 | LCSC136 | A549 |
|---|---|---|---|---|---|
| **133a/133b** | **28** | **6** | **7** | **61** | **9** |
| 1271 | 33 | 23 | 41 | ND | ND |
| **361-3p** | **36** | **27** | **5** | **75** | **11** |
| 1226* | 41 | 43 | 16 | ND | ND |
| 512-5p | 43 | 19 | 23 | ns | 11 |
| 1285 | 45 | 20 | 44 | ND | ND |
| **197** | **46** | **8** | **13** | **80** | **27** |
| Let-7b* | 54 | 11 | 31 | ns | ns |
| 346 | 57 | 25 | 5 | ns | ns |
| 1252 | 62 | ns | 52 | ND | ND |
| 615-3p | 62 | 37 | 68 | ND | ND |
| **636** | **62** | **20** | **37** | **ns** | **37** |
| 1227 | 66 | 41 | ns | ND | ND |
| 885-5p | 73 | ns | 23 | ND | ND |
| 766 | ns | 57 | 43 | ND | ND |
| 1296 | ns | 71 | 48 | ND | ND |
| **204** | **68** | **10** | **47** | **ns** | **30** |
| 663 | ns | 66 | 65 | 86 | ns |
| 191 | ns | 44 | 48 | ns | 64 |

| | | | | | |
|---|---|---|---|---|---|
| ND: not determined ns: not significant | | | | | |

These data indicate that the identified specific inhibitors are able to reduce the viability of all tumor cells tested, including primary cancer stem cells. Thus, these LNAs either neutralize miRNAs or interfere with other pathways important for the general tumor cell survival, at least *in vitro.* The LNAs depicted in table 1 represent valuable tools for targeted anticancer therapy, able to eradicate not only the bulk of differentiated tumor cells, but also drug resistant cancer stem cells, that might be responsible for the frequent relapses after chemotherapy observed in clinics.

### Example 2: Identification of LNAs cytotoxic/cytostatic in breast and lung cancer cell lines.

In the same experimental conditions as above, a series of LNAs have been identified selectively killing all three differentiated cell lines tested but not cancer stem cells (see table 2).

**Table 5: LNAs specifically leading to a decrease in cell viability in cancer cell lines but not in cancer stem cells. For each cancer stem cell and cell line used % of cell viability after LNA transfection is reported**

| hsa-miRNA | MDAMB231 | NIH-H460 | A549 |
|---|---|---|---|
| 574-3p | 15 | 17 | ND |
| 342-3p | 23 | 60 | ND |
| 92a | 24 | 36 | ND |
| 486-5p | 25 | 68 | 44 |
| 150 | 27 | 50 | 27 |
| 210 | 31 | 40 | ns |
| 621 | 33 | 56 | 35 |
| 145 | 34 | 68 | ns |
| 1229 | 38 | 55 | ND |
| 596 | 53 | 55 | 53 |
| 191 | 44 | 48 | 66 |
| 296-5p | 28 | ns | 63 |
| 663 | 66 | 65 | ns |

| | | | |
|---|---|---|---|
| ND: not determined ns: not significant | | | |

The reported LNAs have a high therapeutic value, for example to debulk the main tumor mass as adjuvant treatment before surgery.

### Example 3: synergic effect of LNAs and conventional chemotherapeutic drugs.

LNA-transfected cells were also treated with conventional chemotherapeutic drugs. All LNAs listed in table 1 and table 2 also support the efficiency of chemotherapeutic drugs such as paclitaxel and cisplatin, commonly used to treat patients with breast and lung cancer, respectively.

### Example 4: Identification of LNAs leading to an increase in breast cancer stem cell proliferation.

By applying the same experimental conditions as detailed in example 1, another set of LNAs have been identified as capable to promote the cell growth of the transfected cells. This indicate that the LNAs neutralized miRNAs might act as tumor suppressors. This effect was especially pronounced in the breast cancer stem cell line (BCSC208), in which 11 LNAs led to a significant increase of viability. The data are provided in table 3 that follows.

**Table 6: LNAs leading to increase of breast cancer stem cell proliferation. % of cell viability is reported.**

| hsa-miRNA | BCSC208 | remark |
|---|---|---|
| 206 | 146 | also increases viability of MDA/MB 231 |
| **422a** | **145** | **also increases viability of LCSCs** |
| 33a | 140 | also increases viability of MDA/MB 231 |
| 330-5p | 140 | |
| 526b | 138 | |
| 383 | 137 | |
| 1282 | 136 | |
| 101* | 134 | also increases viability of MDA/MB 231 |
| miR-548 family | 134 | |
| **miR-520 family** | **133** | **also increases viability of LCSC and MDA/MB 231** |
| 370 | 132 | also increases viability of MDA/MB 231 |

The above reported targeted miRNAs might represent tumor suppressor genes which, when overexpressed by using any of the available technologies, can lead to a decrease in proliferation or even to the death of the tumor cells.

### Example 5: Identification of LNAs leading to increased resistance of breast- and lung cancer stem cells and differentiated cell lines to chemotherapeutic treatment.

The genome wide anti miRNA screening detailed in example 1 was then performed after exposure of the indicated cells to chemotherapeutic agents, paclitaxel or cisplatin. A series of LNAs making tumor cells resistant to chemotherapy induced cell death has been identified. These LNAs, listed in table 4, prevented cell death induced by paclitaxel and cisplatin in breast cancer and lung cancer cells, respectively.

**Table 7: LNAs leading to increased resistance of breast- and lung cancer stem cells and differentiated cell lines to chemotherapeutic treatment. % of cell viability is reported.**

| hsa-miRNA | BCSC208 + | MDA/MB-231 + | LCSC136 + | NIH-H460 + |
|---|---|---|---|---|
| | paclitaxel | paclitaxel | cisplatin | cisplatin |
| miR-548 family | 136 | 113 (ns) | 141 | 158 |
| miR-520 family | 138 | 118 (ns) | 122 | ns |
| miR-208a and | 141 | 137 | ns | 172 |
| miR-208b | | | | |
| miR-545-5p and | 139 | ns | ns | 162 |
| miR-545-3p | | | | |
| 196b | 138 | 138 | ns | ns |

| | | | | |
|---|---|---|---|---|
| ns: not significant | | | | |

Increasing the levels of the reported miRNAs is thus functional to avoid the resistance observed following standard chemotherapy.

### Example 6: AntagomiRs approach.

AntagomiR is a synthetic short, single strand RNA conjugated to cholesterol that is perfectly complementary to the specific miRNA target. AntagomiRs are here used as a method to constitutively inhibit the activity of the specific miRNAs identified in the LNA-screens reported in examples 1-4. A cytostatic and cytotoxic activities have been observed with the antagomiR-197 and -663 in lung cancer stem cells (LCSC136) and differentiated lung cancer cell lines (A549 and NIH-H460), respectively. Moreover, antagomiR-361-3p w a s able to substantially induce cell death in breast cancer stem cell clones tested. In addition, antagomiR-361-3p and antagomiR-197 showed only minimal cytotoxic effects on normal, non-transformed cells isolated from breast- and lung tissue, respectively.

## Claims

1. A pharmaceutical composition comprising a neutralizer of the function of at least one of the miRNA selected from the group comprising: hsa-miR-133a (SEQ. ID 1)/133b (SEQ. ID 2), hsa-miR-1271 (SEQ. ID 3), hsa-miR-361-3 (SEQ. ID 4), hsa-miR-1226* (SEQ. ID 5), hsa-miR-512-5p (SEQ. ID 6), hsa-miR-1285 (SEQ. ID 7), hsa-miR-197 (SEQ. ID 8), hsa-miR-Let-7b* (SEQ. ID 9), hsa-miR-346 (SEQ. ID 10), hsa-miR-1252 (SEQ. ID 11), hsa-miR-615-3p (SEQ. ID 12), hsa-miR-636 (SEQ. ID 13), hsa-miR-1227 (SEQ. ID 14), hsa-miR-885-5p (SEQ. ID 15), hsa-miR-766 (SEQ. ID 16), hsa-miR-1296 (SEQ. ID 17), hsa-miR-204 (SEQ. ID 18), hsa-miR-191 (SEQ. ID 19), hsa-miR-663 (SEQ. ID 20), hsa-miR-574-3p (SEQ. ID 21), hsa-miR-342-3p (SEQ. ID 22), hsa-miR-92a (SEQ. ID 23), hsa-miR-486-5p (SEQ. ID 24), hsa-miR-150 (SEQ. ID 25), hsa-miR-210 (SEQ. ID 26), hsa-miR-621 (SEQ. ID 27), hsa-miR-145 (SEQ. ID 28), hsa-miR-1229 (SEQ. ID 29), hsa-miR-596 (SEQ. ID 30), hsa-miR-296-5p (SEQ. ID 31).

2. A pharmaceutical composition comprising an agent capable to increase the function of at least one of the miRNA selected from the group comprising: hsa-miR-206 (SEQ. ID 32), hsa-miR-422a (SEQ. ID 33), hsa-miR-33a (SEQ. ID 34), hsa-miR-330-5p (SEQ. ID 35), hsa-miR-526b (SEQ. ID 36), hsa-miR-383 (SEQ. ID 37), hsa-miR-1282 (SEQ. ID 38), hsa-miR-101* (SEQ. ID 39), hsa-miR-548 family (SEQ. ID 40), hsa-miR-520 family (SEQ. ID 41), hsa-miR-370 (SEQ. ID 42), hsa-miR-208a (SEQ. ID 43) and hsa-miR-208b (SEQ. ID 44), hsa-miR-545-3p (SEQ. ID 45) and hsa-miR-545-5p (SEQ. ID 46), hsa-miR-196b (SEQ.
ID 47).

3. The pharmaceutical composition according to claim 1 or 2 which comprises in addition a conventional chemotherapeutic drugs, preferably paclitaxel or cisplatin.

4. The pharmaceutical composition according to any of the claims 1 to 3 for use in cancer therapy, wherein said cancer is preferably breast or lung cancer.

5. A method of reducing the proliferation of a cancer cell which comprises neutralizing the function of at least one of the miRNA selected from the group comprising: hsa-miR-133a (SEQ. ID 1)/133b (SEQ. ID 2), hsa-miR-1271 (SEQ. ID 3), hsa-miR-361-3 (SEQ. ID 4), hsa-miR-1226* (SEQ. ID 5), hsa-miR-512-5p (SEQ. ID 6), hsa-miR-1285 (SEQ. ID 7), hsa-miR-197 (SEQ. ID 8), hsa-miR-Let-7b* (SEQ. ID 9), hsa-miR-346 (SEQ. ID 10), hsa-miR-1252 (SEQ. ID 11), hsa-miR-615-3p (SEQ. ID 12), hsa-miR-636 (SEQ. ID 13), hsa-miR-1227 (SEQ. ID 14), hsa-miR-885-5p (SEQ. ID 15), hsa-miR-766 (SEQ. ID 16), hsa-miR-1296 (SEQ. ID 17), hsa-miR-204 (SEQ. ID 18), hsa-miR-191 (SEQ. ID 19), hsa-miR-663 (SEQ. ID 20), hsa-miR-574-3p (SEQ. ID 21), hsa-miR-342-3p (SEQ. ID 22), hsa-miR-92a (SEQ. ID 23), hsa-miR-486-5p (SEQ. ID 24), hsa-miR-150 (SEQ. ID 25), hsa-miR-210 (SEQ. ID 26), hsa-miR-621 (SEQ. ID 27), hsa-miR-145 (SEQ. ID 28), hsa-miR-1229 (SEQ. ID 29), hsa-miR-596 (SEQ. ID 30), hsa-miR-296-5p (SEQ. ID 31).

6. The method of claim 5, wherein said neutralization of miRNA function is obtained by one of the available technologies, preferably selected from a a locked nucleic acid (LNA) oligo, a Morpholino oligo or a 2'-O-methyl RNA oligo.

7. The method of claim 5, wherein said neutralization of miRNA function is obtained by a complementary antagomirR or specific steric-blocking oligos.

8. The method of claim 5, wherein said miRNA is selected from the group comprising hsa-miR-133a (SEQ. ID 1)/133b (SEQ. ID 2), hsa-miR-1271 (SEQ. ID 3), hsa-miR-361-3 (SEQ. ID 4), hsa-miR-1226* (SEQ. ID 5), hsa-miR-512-5p (SEQ. ID 6), hsa-miR-1285 (SEQ. ID 7), hsa-miR-197 (SEQ. ID 8), hsa-miR-Let-7b* (SEQ. ID 9), hsa-miR-346 (SEQ. ID 10), hsa-miR-1252 (SEQ. ID 11), hsa-miR-615-3p (SEQ. ID 12), hsa-miR-636 (SEQ. ID 13), hsa-miR-1227 (SEQ. ID 14), hsa-miR-885-5p (SEQ. ID 15), hsa-miR-766 (SEQ. ID 16), hsa-miR-1296 (SEQ. ID 17), hsa-miR-204 (SEQ. ID 18) and said cancer cell is a cancer stem cell and/or a differentiated carcinoma cell, preferably from breast or lung tumors.

9. The method of claim 5, wherein said miRNA is selected from the group comprising hsa-miR-574-3p (SEQ. ID 21), hsa-miR-342-3p (SEQ. ID 22), hsa-miR-92a (SEQ. ID 23), hsa-miR-486-5p (SEQ. ID 24), hsa-miR-150 (SEQ. ID 25), hsa-miR-210 (SEQ. ID 26), hsa-miR-621 (SEQ. ID 27), hsa-miR-145 (SEQ. ID 28), hsa-miR-1229 (SEQ. ID 29), hsa-miR-596 (SEQ. ID 30), hsa-miR-191 (SEQ. ID 19), hsa-miR-296-5p (SEQ. ID 31), hsa-miR-663 (SEQ. ID 20) and said cancer cell is a differentiated carcinoma cell, preferably from breast or lung tumors.

10. A method of reducing the proliferation of a cancer cell
which comprises increasing the function of at least one of the miRNA selected from the group comprising: hsa-miR-206 (SEQ. ID 32), hsa-miR-422a (SEQ. ID 33), hsa-miR-33a (SEQ. ID 34), hsa-miR-330-5p (SEQ. ID 35), hsa-miR-526b (SEQ. ID 36), hsa-miR-383 (SEQ. ID 37), hsa-miR-1282 (SEQ. ID 38), hsa-miR-101* (SEQ. ID 39), hsa-miR-548 family (SEQ. ID 40), hsa-miR-520 family (SEQ. ID 41), hsa-miR-370 (SEQ. ID 42).

11. The method of claim 10, wherein said increase is accomplished through the introduction of a synthetic miRNA mimic into the cytoplasm that can be processed and loaded into miRNA RNA induced silencing complex (miRISC) by Dicer.

12. The method of claim 10, wherein said increase is accomplished through the expression of at least one of said miRNAs from RNA polymerase (Pol) III promoters as an artificial short hairpin RNA (shRNA).

13. The method of claim 10, wherein said increase is accomplished through the expression of at least one of said miRNAs from an RNA Pol II promoter.

14. A method to prevent the resistance of tumor cells to
chemotherapeutic agents which comprises increasing the function of at least one of the miRNA selected from the group comprising: hsa-miR-548 family (SEQ. ID 40), hsa-miR-520 family (SEQ. ID 40), hsa-miR-208a (SEQ. ID 43) and hsa-miR-208b (SEQ. ID 44), hsa-miR-545-3p (SEQ. ID 45) and hsa-miR-545-5p (SEQ. ID 46), hsa-miR-196b (SEQ. ID 47).

15. The method of claim 14, wherein said tumor cells cell are preferably breast cancer cells and said chemotherapeutic agent is paclitaxel.

16. The method of claim 14, wherein said tumor cells are
preferably lung cancer cells and said chemotherapeutic agent is cisplatin.
